# NOUVEAU FASCICULE DE BREVET EUROPEEN

(11) **EP 0 930 936 B2**
(45) Date de publication et mention de la décision concernant l'opposition: **27.08.2003**
(45) Mention de la délivrance du brevet: 26.07.2000
(21) Numéro de dépôt: 97942080.9
(22) Date de dépôt: 24.09.1997
(51) Int. Cl.: B01J 13/02

(54) **PROCEDE DE PREPARATION DE MICROCAPSULES DE MATIERES ACTIVES ENROBEES PAR UN POLYMERE ET NOUVELLES MICROCAPSULES NOTAMMENT OBTENUES SELON LE PROCEDE**
VERFAHREN ZUR HERSTELLUNG VON AKTIVE STOFFE ENTHALTENDEN MIKROKAPSELN UND MIT EINEM POLYMER UMGEHÜLLTEN
METHOD FOR PREPARING MICROCAPSULES OF ACTIVE SUBSTANCES COATED WITH A POLYMER AND NOVEL MICROCAPSULES IN PARTICULAR RESULTING FROM THE METHOD

(30) Priorité: 25.09.1996 FR 9611665
(43) Date de publication de la demande: 28.07.1999
(73) Titulaire: MAINELAB, 49100 Angers (FR)
(72) Inventeur: BENOIT, Jean-Pierre, F-49240 Avrille (FR); RICHARD, Jo[l, F-49160 Longue (FR); THIES, Curt, Ballwin, MO 6301 (US)
(74) Mandataire: Ahner, Francis
(86) Numéro de dépôt international: FR9701674
(87) Numéro de publication internationale: WO98013136

(56) Documents cités:
- EP-A- 0 322 687
- EP-A- 0 542 314
- EP-A- 0 706 821
- WO-A-90/03782
- WO-A-92/11000
- WO-A-95/01221
- WO-A-96/00610
- WO-A-96/11055
- WO-A-97/03657
- WO-A-97/31691
- "Handbook of Pharmaceutical Excipients (American Pharmac. Association & The Pharmac. Press)", deuxième édition (1994), pages v-vii, 186, 223-225, 229, 362 et 363. (ISBN (UK) 0-85369-305-6)
- "Microencapsulation"" édité par J.R.Nixon, Edition Marcel Dekker Inc. (1996), pages 2 et 3, (ISBN (US) 0-8247-6338-6)
- "Gas Antisolvent Recrystallisation: New Process to Recrystallize Compounds Insoluble in Supercritical Fluids" par P.M.Gallagher et al., ACS Symposium Series 406, American Chemical Society (1989), Chapitre 22, pages 334-353

## Description

L'invention concerne le domaine de la microencapsulation de substances actives. Elle est relative à un procédé de préparation de microcapsules du type comprenant une substance active enrobée d'une couche de polymère par la technique dite du fluide supercritique.

Elle concerne également de nouvelles microcapsules contenant notamment des substances pharmaceutiques, cosmétiques ou agroalimentaires.

De très nombreux documents décrivent les principes et les méthodes de préparation de telles microcapsules, par exemple J.A. Bakan, Microencapsulation via Coacervation - Phase Separation, National Industrial Research Conference, Land O'lakes, Wl, Juin 1966. D'autres publications sont citées dans l'article de J.P. Benoit et al., Microencapsulation Methods and Industrial Applications, Marcel Dekker, Inc. 1996, pages 35-72.

La taille des microcapsules obtenues est de l'ordre de 0,5 à 200µm (parfois davantage). Elles sont constituées d'un noyau de matière active revêtu d'un agent enrobant.

L'agent enrobant est choisi parmi un éventail de divers composés (hydrocolloïdes, polymères hydrophobes, cires, graisses ou agents entériques etc.) selon divers facteurs :
- les buts recherchés par la microencapsulation : par exemple, masquer le goût ou l'odeur du principe actif per os, diminuer la volatilité de certains liquides, augmenter la stabilité physico-chimique du principe actif, prévenir la coalescence des gouttelettes au sein d'une émulsion, modifier et améliorer la compression du principe actif, retarder ou prolonger l'action d'un médicament, avoir une forme galénique entérosoluble etc.
- le mode de libération désiré du principe actif (dissolution, diffusion...)
- la nature physico-chimique du noyau (dimension, compatibilité....),
- les méthodes de microencapsulation (fluidisation, turbine, dessiccation par nébulisation, polymérisation interfaciale, coacervation...).

La préparation des microcapsules est couramment mise en oeuvre par la méthode dite de coacervation que l'on rappelle sommairement ci-après:

Par modification chimique ou physico-chimique d'un milieu contenant une substance active en suspension dans une solution de polymère dans un solvant, on provoque la coacervation (ou agrégation) du polymère.

Les gouttelettes de coacervat ainsi formées sont adsorbées à la surface des particules de matière active et forment un enrobage continu.

On soumet ensuite le milieu à une désolvatation complète et éventuellement une réticulation des chaînes polymériques, qui conduit à la production de microcapsules.

Toutes ces méthodes, citées ci-dessus, requièrent bien souvent des solvants organiques toxiques, polluants et d'un coût industriel relativement élevé.

C'est pourquoi, la Demanderesse s'est attachée depuis plusieurs années à rechercher des procédés alternatifs qui permettent de préparer des microcapsules sans l'aide de solvants organiques ou du moins sans solvants chlorés (dichlorométhane, chloroforme, etc.).

C'est ainsi que la demanderesse a récemment proposé par la demande de brevet EP-A-706 821 d'utiliser les propriétés du CO₂ à l'état supercritique (SC) comme solvant.

Le CO₂ est dit à l'état supercritique (CO₂ SC) si la température est supérieure à 31°C et sa pression supérieure à 73,8.10⁵ Pa.

En effet, ce dernier garde à la fois les propriétés d'un gaz, telle sa grande diffusion, et acquiert celles d'un liquide, telle sa densité qui est de 0,7 kg/cm³ au point supercritique. Le CO₂, dans cet état, possède un pouvoir solvant notable. On le dit semblable à l'heptane.

Les avantages du CO₂ SC sont donc :
- un grand pouvoir solvant pour des conditions de température assez basses (30°C), ce qui n'est pas dénué d'intérêt dans le cas d'utilisation de principes actifs thermolabiles ;
- une grande variation de ce pouvoir solvant pour de faibles variations de pression,
- sa non toxicité,
- la séparation facile du mélange solvant-soluté par simple décompression ;
- son coût peu onéreux par rapport aux solvants organiques courants.

Le procédé selon la demande de brevet précitée implique la mise en suspension dans un autoclave d'une substance active non soluble dans le CO₂ supercritique, puis l'introduction dans l'autoclave de l'enrobant qui se trouve à l'état de soluté dans le CO₂ supercritique. La pression et/ou la température sont ensuite modifiées de telle manière que la solubilité de l'enrobant dans le CO₂ diminue. L'affinité de l'enrobant pour la surface de la substance active s'accroît provoquant de ce fait l'adsorption de l'enrobant autour des particules de substance active. Une fois le dépôt accompli, l'autoclave est dépressurisé et les microcapsules récupérées. Ce procédé donne d'excellents résultats pour les agents enrobants qui présentent une bonne solubilité dans le CO₂, c'est-à-dire pour les agents enrobants qui possèdent un caractère lipophile marqué et de faibles masses molaires comme les corps gras (cires, triglycérides d'alcools gras, acides gras) et bien d'autres composés.

Toutefois, dans le cas de polymères qui présentent un caractère sensiblement plus polaire que les corps gras et une masse molaire plus élevée (polymères acryliques, polymères vinyliques, polysaccharides) ce procédé est peu satisfaisant.

En effet, ces polymères ne sont pas solubles dans le CO₂ supercritique. Or, il s'agit d'une condition impérative pour être un agent enrobant selon le procédé décrit dans la demande de brevet EP-A-706 821. On a bien envisagé dans cette demande de brevet d'utiliser à faible taux ( < 5 %) un agent entraînant comme les cétones, les alcools, les esters et les solvants chlorés en mélange avec le CO₂ dans le but d'augmenter la solubilité du polymère dans la phase supercritique. Toutefois, une telle variante conduirait néanmoins, dans bien des cas, à un taux de solubilisation faible du polymère à caractère sensiblement polaire et à une modification importante des conditions supercritiques, ou à une disparition de la phase supercritique remplacée par un système biphasique.

On cite par ailleurs, le document US 5 424 076 A (WO 92 11 000 A) qui décrit un procédé basé sur une technique d'atomisation ou spray-drying en présence de fluide supercritique.

En premier lieu, bien que le document mentionne le fait que la substance active soit sous forme dispersée, tous les exemples sont relatifs à des solutions de ces matières actives. Par ailleurs, ce procédé conduit à l'obtention de microsphères et non pas de microcapsules. On rappelle la différence fondamentale entre les microsphères et les microparticules. Les microsphères sont des systèmes matriciels dans lesquels la matière active est dispersée de façon homogène. Les microcapsules sont constituées d'un noyau de matière active enrobé d'une couche de polymère.

Le document EP-A-542 314 est relatif à un procédé de préparation de particules de matière active sans enrobage de polymère par précipitation grâce à un fluide antisolvant à l'état supercritique.

L'objet de la présente invention est donc en premier lieu de proposer un nouveau procédé faisant appel à un fluide supercritique qui permet l'obtention de microcapsules dont l'agent enrobant est un polymère à caractère sensiblement polaire.

Un autre objet de la présente invention est de proposer de nouvelles microcapsules qui se distinguent notamment de celles qui ont été précédemment décrites par leur caractéristiques physiques et par l'absence de solvant résiduel dans la couche d'enrobage.

L'invention concerne donc en premier lieu un procédé de préparation de microcapsules comprenant une substance active enrobée d'une couche de polymère polaire, caractérisé en ce qu'il comprend les étapes suivantes :
- mettre en suspension une substance active dans une solution de polymère sensiblement polaire dans un solvant organique, la substance active étant insoluble dans ce solvant.
   ledit polymère sensiblement polaire étant insoluble dans le CO₂ liquide ou à l'état supercritique,
   ledit solvant organique étant soluble dans le CO₂ liquide ou supercritique.
- mettre en contact la suspension avec du CO₂ liquide ou supercritique par introduction du CO₂ dans un réacteur fermé contenant déjà la suspension de façon à désolvater de façon contrôlée le polymère sensiblement polaire dans des conditions voisines de l'équilibre, sans brusques variations de pression (détente),
- assurer sa coacervation,
- extraire substantiellement le solvant au moyen de CO₂ à l'état supercritique et évacuer le mélange CO₂/solvant, si nécessaire par plusieurs cycles d'introduction de CO₂ suivi de pompage,
- récupérer les microcapsules dans l'autoclave sous forme de poudre sèche.

Par l'expression "de façon contrôlée", on entend le fait que le système est toujours dans des conditions voisines de l'équilibre et ne subit pas de brusques variations de pression (détente).

On remarquera que ce procédé se différencie radicalement du procédé décrit dans la demande de brevet européen EPO 706 821 A dans la mesure où le polymère n'est à aucun moment en solution dans le fluide à l'état liquide ou supercritique.

Par ailleurs, ce procédé présente une simplification notable par rapport aux procédés classiques (émulsion-évaporation de solvant)du fait de l'élimination de la phase de séchage généralement longue et difficile (10 à 15 jours sous vide dynamique 10 Pa).

### Le fluide supercritique

Bien que l'invention ait plus particulièrement pour objet un procédé faisant intervenir le CO₂ en tant que fluide, le procédé peut être étendu à d'autres fluides dont le comportement est connu pour être similaire au CO₂ comme ceux cités par J.P. Benoit et al (op-cit). Néanmoins, ce procédé sera plus particulièrement décrit dans le cas du CO₂.

### L'agent enrobant

Les polymères à caractère sensiblement polaire relatifs à l'invention seront plus particulièrement choisis parmi :
1) les polysaccharides et leurs dérivés tels que :
   - l'amidon ou l'amidon modifié comme les carboxyméthylamidons, les polysaccharides résultant de la dépolymérisation par une méthode physique, chimique ou enzymatique de l'amidon ou ses dérivés,
   - la cellulose ou la cellulose modifiée comme les carboxyméthylcelluloses, l'éthylcellulose, l'hydroxyméthylcellulose, l'hydroxyéthylcellulose, l'hydroxypropylcellulose, la méthylhydroxyéthylcellulose, la méthylhydroxypropylcellulose, les polysaccharides résultant de la dépolymérisation par une méthode physique, chimique ou enzymatique de la cellulose ou ses dérivés,
   - les alginates extraits d'algues brunes,
   - les carraghénanes de type lambda, iota ou kappa extraits d'algues rouges,
   - les pectines extraites des citrons, des pommes ou des betteraves,
   - les pectates qui résultent de la déméthylation des pectines,
   - les guars, les guars modifiées tels que les carboxyméthylguars,
   - les xanthanes,
   - les chitosans,
2) Les polymères synthétiques du type acrylique ou méthacrylique comme les homopolymères ou les copolymères de l'acide acrylique ou méthacrylique, des esters de l'acide acrylique ou méthacrylique, le polyacrylamide, les polycyanoacrylates et en général tous les polymères synthétiques bien connus dérivés de l'acide acrylique ou méthacrylique, les polymères et copolymères vinyliques dérivés des esters vinyliques (polyacétate de vinyle), les copolymères de l'éthylène et de l'acétate de vinyle.
   Ces polymères ne sont pas solubles dans le fluide à l'état liquide ou supercritique, notamment le CO₂.
   On mentionnera tout particulièrement les polymères du type de ceux commercialisés sous la marque Eudragit® par la société RÖHM tels que les copolymères d'esters acryliques ou méthacryliques neutres issus de la déshydratation de dispersions aqueuses (Eudragit® NE 30D et NE 40D), les copolymères d'esters acryliques ou méthacryliques porteurs de groupements ammonium quaternaires (Eudragit® RL 100 et RS 100). les copolymères d'esters acryliques ou méthacryliques porteurs de fonctions amines (Eudragit® E 100) ou enfin les copolymères d'esters acryliques ou méthacryliques porteurs de groupements carboxyliques (Eudragit® L 100 et S 100).
3) Les polymères et copolymères biodégradables des acides α-hydroxycarboxyliques. notamment les homopolymères et copolymères des acides lactiques et glycoliques,
4) La poly (ε-caprolactone) et ses dérivés, les poly (β-hydroxybutyrate, poly(hydroxyvalerate) et les copolymères (β-hydroxybutyrate-hydroxyvalérate), le polyacide malique.
5) Les polymères-blocs amphiphiles de type polyacide lactique-polyoxyde d'éthylène.
6) Les polycondensats tels que les polyamides et les polyesters (polyéthylène téréphtalate) et les polymères issus de polyaddition (polydiméthyl siloxane).
7) Les polyanhydrides, les polyorthoesters et les polyphosphazènes.

Ces polymères choisis pour être des agents enrobants efficaces présentent une masse molaire supérieure à 10³ g/mole, de préférence supérieure à 2 x 10³ g/mole et plus particulièrement comprise entre 2.10³ et 2 x 10⁵ g/mole.

### La substance active

La substance active doit être insoluble dans le solvant organique. Parmi les nombreuses substances qui peuvent être enrobées, on peut citer à titre non limitatif :
* Produits pharmaceutiques :
   - antalgiques (paracétamol notamment)
   - antipyrétiques
   - aspirine et dérivés
   - antibiotiques
   - anti-inflammatoires
   - anti-ulcéreux
   - anti-hypertenseurs
   - neuroleptiques
   - anti-dépresseurs
   - oligonucléotides
   - peptides
   - protéines
* Produits cosmétiques :
   - auto-bronzant, anti-UV
* Produits agro-alimentaires:
   - vitamines

Ces substances actives se présentent sous la forme de poudre de faible granulométrie, typiquement de l'ordre de quelques micromètres, et plus généralement de 0,1 µm à 800 µm.

Une caractéristique remarquable du procédé selon l'invention est qu'il peut être mis en oeuvre à partir de particules de matières actives de géométrie très variée, comprenant aussi bien des formes très régulières ou au contraire très irrégulières. Le procédé peut être utilisé pour encapsuler des particules parfaitement sphériques, des cristaux ou microcristaux non sphériques mais présentant des formes très régulières, ou encore des particules de forme très irrégulière. Ce peuvent être par exemple des poudres obtenues par cristallisation, précipitation, pyrolyse, évaporation d'une solution, atomisation-séchage, mais aussi des poudres issues d'un broyage, d'une granulation, d'un procédé d'extrusion, ou de tout procédé mécanique de réduction de taille. Des particules solides poreuses peuvent également être encapsulées par ledit procédé. Dans ce cas, une des caractéristiques de l'enrobage réside dans son aptitude à épouser fidèlement la surface des particules jusque dans les pores et les crevasses de la surface, car la désolvatation du polymère et sa précipitation ou condensation à la surface des particules est bien contrôlée par la quantité et les conditions d'introduction (pression, température) du CO₂, la microcapsule finale est alors caractérisée par l'absence de pores en surface susceptibles d'exposer la matière active au milieu extérieur.

### Le solvant du polymère

Le choix du solvant ou mélange de solvants convenant dans le cadre du procédé selon l'invention est fonction de la nature du polymère à mettre en solution.

Dans le cas des polymères acryliques, on citera les cétones (cyclohexanones) les alcools (méthanol. éthanol. butanol. isopropanol, propylène glycol), les mélanges eau/alcool, acétone/alcool, le P. G.A. (acétate de polypropylène glycol), les esters (acétate d'éthyle).

Le solvant présente généralement un caractère polaire protique ou aprotique et n'est pas un agent entraîneur du polymère dans le CO₂, i.e. il n'augmente pas de façon significative la solubilité du polymère dans le CO₂ supercritique.

### Principe du procédé

Sur un plan pratique, le procédé est mis en oeuvre de préférence sous agitation dans un réacteur fermé notamment un autoclave. La mise en contact du CO₂ avec la suspension de principe actif contenant le polymère en solution est effectuée de façon contrôlée dans des conditions voisines de l'équilibre, sans brusques variations de pression (détente), par introduction du CO₂ dans un réacteur fermé notamment un autoclave contenant déjà la suspension.

On rappelle que ce contact intime entre le CO₂ et la suspension est assuré soit avec un CO₂ sous forme liquide/gaz (le liquide étant mélangé à la suspension et mouillant les particules de substance active), soit directement par du CO₂ à l'état supercritique.

Selon une variante, la suspension est mise en contact avec du CO₂ liquide, puis l'on augmente la pression et/ou la température de façon à faire passer le CO₂ à l'état supercritique pour extraire le solvant. De préférence, la température du CO₂ liquide est comprise entre 20 et 50°C et la pression entre 50.10⁵ et 150.10⁵ Pa. De préférence, la température du CO₂ supercritique est comprise entre 35 et 45°C et la pression entre 100.10⁵ et 140.10⁵ Pa.

Le poids de solvant du polymère introduit dans le réacteur fermé notamment l'autoclave représente au moins 3 % du poids de fluide, supercritique ou liquide, utilisé pour provoquer la désolvatation du polymère et préférentiellement entre 3,5 % et 25 % du poids du fluide.

Il se produit une séparation de phase avec coacervation (précipitation) du polymère autour des particules de substance active et passage du solvant dans la phase CO₂.

L'invention concerne également de nouvelles microcapsules comprenant une substance active enrobée dans une couche de polymère sensiblement polaire, caractérisées en ce que la couche de polymère présente une faible énergie de surface et en ce que les microcapsules sont susceptibles d'être obtenues par le procédé selon l'invention.

Selon des caractéristiques supplémentaires, la couche de polymère épouse la surface de la particule de substance active jusque dans les porosités internes et les microcapsules présentent une surface exempte de pores exposant la particule de substance active au milieu extérieur.

Ces microcapsules sont en effet nouvelles en soi pour les deux raisons suivantes. En effet, en premier lieu, la couche d'enrobage présente une conformation différente à plusieurs titres. Elle est caractérisée par l'absence de pores en surface susceptibles d'exposer la matière active au milieu extérieur, et par l'absence substantielle de motifs polaires à la surface de la microcapsule. Pour cette raison, ces microcapsules diffèrent de celles obtenues par le procédé de coacervation classique tel qu'il a été décrit ci-dessus dans la description. L'origine de cette conformation différente est liée au fait que la désolvatation est effectuée de façon contrôlée grâce à l'apport d'une quantité de CO₂ appropriée, dans des conditions de pression et température bien choisies et que le CO₂ présente un caractère peu polaire, qui induit l'orientation vers la surface des particules des groupements du polymère les moins polaires.

Un autre aspect remarquable des microcapsules selon l'invention est que la couche d'enrobage est quasiment exempte de solvant. En tout cas, elle contient moins de 500 ppm de solvant et de préférence moins de 300 ppm de solvant.

Ces nouvelles microcapsules présentent par ailleurs, du fait de leur faible énergie de surface, un effet barrière plus important, qui de ce fait ralentit la dégradation de la couche d'enrobage et la diffusion de la substance active vers les milieux polaires tels que les fluides physiologiques, les formulations aqueuses, .... La dimension de ces microcapsules varie entre 10 nm et 1 mm, et préfentiellement entre 20 nm et 500 µm.

Le taux de substance active est avantageusement compris entre 25 et 95 % (en poids), et préférentiellement entre 60 % et 90 % (en poids).

L'invention concerne également l'application de ces microcapsules à la réalisation de produits cosmétiques, pharmaceutiques ou agroalimentaires.

L'invention va maintenant être décrite par les exemples non limitatifs ci-après.

### Exemple 1

On solubilise 40 mg d'un copolymère acide méthacrylique. acrylate et méthacrylate d'alkyle commercialisé par la société Röhm sous la marque Eudragit L. 100® dans 54 ml d'éthanol absolu. On met 200 mg d'hémoglobine bovine (HB) provenant de la Société Sigma, en suspension dans la solution ainsi obtenue et on place la suspension dans un autoclave de capacité 1,5 l.

Dans un premier temps, on monte en pression à 80.10⁵ Pa en introduisant le CO₂ liquide tout en restant à température constante de 25°C. Le CO₂ se maintient donc à l'état liquide.

Le CO₂ liquide se mélange avec la suspension permettant de mouiller convenablement l'hémoglobine. Le CO₂ liquide assure la précipitation progressive du polymère. On fait passer le CO₂ à l'état supercritique en augmentant la pression jusqu'à 125.10⁵ Pa et en montant conjointement la température à 40°C (ce qui correspond à une densité de CO₂ d'environ 0,72 kg/dm³), ce qui permet d'extraire l'éthanol. On maintient ces conditions pendant 15 minutes. On évacue le mélange CO₂/éthanol, en décompressant jusqu'à 75.10⁵ Pa (afin de rester en phase supercritique) dans le séparateur, au niveau duquel l'éthanol est récupéré et le CO₂ retourne dans un réservoir. 25 ml d'éthanol sont récupérés. On réitère plusieurs cycles successifs d'introduction du CO₂ liquide, de passage à l'état supercritique et d'évacuation du mélange CO₂/ethanol jusqu'à élimination complète de l'éthanol. La décompression se fait obligatoirement par la phase gazeuse afin de ne pas reconcentrer le polymère dans l'éthanol restant.

Après la phase de décompression, on peut ainsi répéter l'opération plusieurs fois en réintroduisant du CO₂ afin de retrouver une pression de 125.10⁵ Pa et une température de 40°C.

Ensuite, on peut dépressuriser et relâcher le mélange CO₂/solvant à l'extérieur puis introduire du CO₂ frais que l'on porte à l'état supercritique afin d'extraire complètement le solvant. La température dans ce cas est généralement comprise entre 35 et 45°C et la pression entre 100 et 140.10⁵ Pa.

Selon une variante, on peut également directement mettre en contact la suspension avec du CO₂ supercritique dans les conditions indiquées ci-dessus.

230 mg de microcapsules de taille moyenne 200 à 300 µm et comportant 83,3 % en poids d'hémoglobine sont récupérées et il ne reste plus d'éthanol dans l'autoclave. Les microcapsules résistent à l'eau. En effet, on voit nettement, grâce à une observation microscopique, que l'hémoglobine enrobée ne se dissout pas dans une goutte d'eau, alors qu'un échantillon non enrobé donne rapidement une coloration rouge à la goutte d'eau.

### Exemples 2-8:

Plusieurs autres essais effectués dans les mêmes conditions, mais en faisant varier la quantité d'hémoglobine bovine (HB) et la quantité de solvant, ont donné les résultats suivants:

| ex. | eth(ml) | HB(mg) | HB/Eudr(mg) | HB/Eudr |
|---|---|---|---|---|
| 2 | 50 | 500 | 500/99,9 | 83,31/16,7 |
| 3 | 50 | 515 | 515/50,31 | 91,1/8,9 |
| 4 | 50 | 511,5 | 511,5/158 | 76,4/23,6 |
| 5 | 50 | 1012,5 | 1012,5/103 | 90,8/9,2 |
| 6 | 50 | 233,4 | 233,4/100 | 70/30 |
| 7 | 50 | 501 | 501/101 | 83,2/16,8 |
| 8 | 50 | 502 | 502/101 | 83,2/16,8 |
| eth : éthanol | | | | |
| HB : hémoglobine bovine | | | | |
| Eudr : Eudragit L100® | | | | |

### Vérification de la qualité de l'enrobage

On compare la cinétique de dissolution de l'hémoglobine microencapsulée dans un tampon phosphate de pH = 7 durant une heure, à 37°C par rapport à de l'hémoglobine non encapsulée (échantillon témoin T). Dans un récipient muni d'un agitateur sur la tige duquel est attaché un sachet en papier contenant l'échantillon à tester, on effectue des prélèvements au cours du temps et l'on évalue la concentration de l'échantillon par la spectrophotométrie dans le visible (405 nm = λₘₐₓ de l'hémoglobine). On remarque que l'Eudragit L-100 n'absorbe pas à cette longueur d'onde.

La cinétique de dissolution est comparée à celle obtenue pour de l'hémoglobine non traitée.

Les résultats sont indiqués dans le tableau ci-dessous après une heure de dissolution.

| Echantillon | concentration en HB (g/l) |
|---|---|
| Témoin T (taux de charge 100 %) | 0.1763 |
| 2 | 0.0343 |
| 3 | 0.019 |
| 4 | 0.0414 |
| 5 | 0.0048 |
| 6 | 0.0009 |
| 7 | 0,0286 |
| 8 | 0,0323 |

On constate donc un fort retard à la libération du fait de l'enrobage.

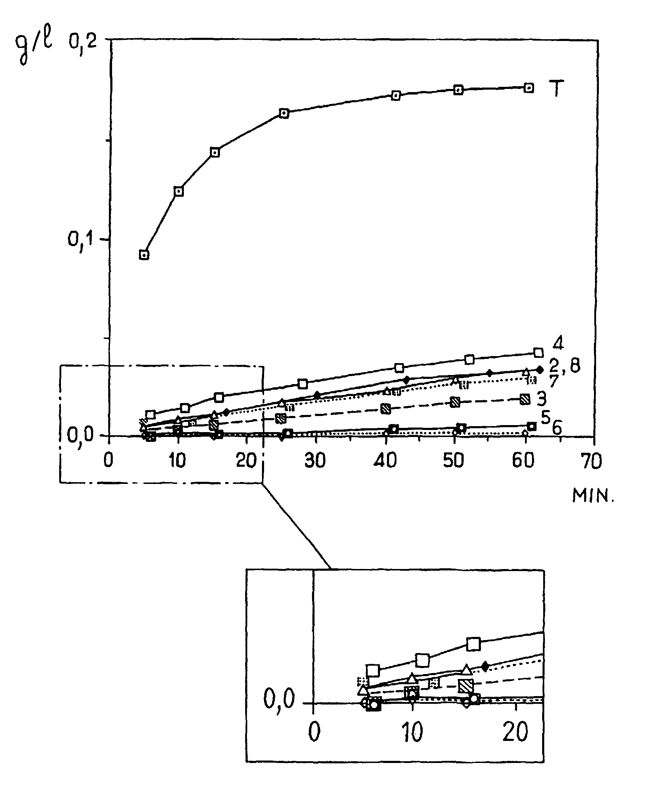

Ces mesures de cinétique sont représentées sur la figure annexée (concentration d'hémoglobine/temps). Un retard à la dissolution est observé de manière nette.

### Exemple 9

On solubilise 4 g d'homopolymère d'acide L lactique (PLA) Résomer® L 206 (commercialisé par BOEHRIN-GER-INGELHEIM) dans 130 ml de dichlorométhane. On met 8 g de poudre d'albumine de sérum bovin (BSA) (commercialisée par la société SIGMA) en suspension dans la solution ainsi obtenue.

On monte en pression à 80.10⁵ Pa en introduisant le CO₂ liquide, la température étant maintenue à 25°C. Le CO₂ est ainsi maintenu à l'état liquide.

Le CO₂ liquide se mélange à la suspension et provoque la précipitation progressive du polymère. On fait passer le CO₂ à l'état supercritique en augmentant la pression à 90.10⁵ Pa et en montant conjointement la température à 40°C, ce qui permet d'extraire le dichlorométhane.

On maintient ces conditions pendant 30 min. On évacue le mélange CO₂/dichlorométhane vers le séparateur au niveau duquel le dichlorométhane est récupéré, et le CO₂ recyclé retourne dans le réservoir. On réitère plusieurs cycles successifs d'introduction du CO₂, de passage à l'état supercritique et d'évacuation du mélange CO₂/dichlocométhane jusqu'à élimination complète du dichlorométhane.

On récupère dans l'autoclave 11,2 g de microcapsules sous forme de poudre sèche d'une taille moyenne de 50 µm environ. Un dosage du dichlorométhane par chromatographie en phase gaz effectué après hydrolyse basique (Na OH 1N) en présence de chlorure d'isopropyle comme étalon interne montre que le taux de dichlorométhane résiduel dans les microcapsules est inférieur ou égal à 300 ppm (par rapport au polymère).

Par ressolubilisation des microcapsules obtenues dans le dichlorométhane, on détermine le taux de BSA contenue dans ces capsules : ce taux est trouvé de 68 % (en poids, par rapport au poids total de microcapsules).

## Revendications

1. Procédé de préparation de microcapsules comprenant une substance active enrobée d'une couche de polymère sensiblement polaire, **caractérisé en ce qu'**il comprend les étapes suivantes :
- mettre en suspension une substance active dans une solution de polymère sensiblement polaire dans un solvant organique, ladite substance étant insoluble dans le solvant organique,
ledit polymère sensiblement polaire étant insoluble dans le CO₂ liquide ou à l'état supercritique,
ledit solvant organique étant soluble dans le CO₂ liquide ou supercritique,
- mettre en contact la suspension avec du CO₂ liquide ou supercritique par introduction du CO₂ dans un réacteur fermé contenant déjà la suspension de façon à désolvater de façon contrôlée le polymère sensiblement polaire dans des conditions voisines de l'équilibre, sans brusques variations de pression (détente),
- assurer sa coacervation,
- extraire substantiellement le solvant au moyen du CO₂ à l'état supercritique et évacuer le mélange CO₂/solvant,
- récupérer les microcapsules.

2. Procédé de préparation de microcapsules selon la revendication 1, **caractérisé en ce que** le polymère est choisi dans le groupe constitué par les polysaccharides, les dérivés cellulosiques, les polymères dérivés de l'acide acrylique ou méthacrylique, les polymères biodégradables de type poly(α-hydroxyacide), les polymères dérivés des esters vinyliques, les polyesters, les polyamides, les polyanhydrides, les polyorthoesters, les polycyanoacrylates, les polyphosphazènes.

3. Procédé de préparation de microcapsules selon la revendication 2, **caractérisé en ce que** les polymères présentent une masse molaire comprise entre 2.10³ et 2.10⁵ g/mole.

4. Procédé de préparation de microcapsules selon la revendication 1, **caractérisé en ce que** le solvant est choisi dans le groupe constitué par les cétones, les alcools, les esters.

5. Procédé de préparation de microcapsules selon l'une des revendications 1 à 4, **caractérisé en ce que** la suspension est mise en contact avec du CO₂ liquide, puis **en ce que** l'on augmente la pression et/ou la température de façon à faire passer du CO₂ à l'état supercritique pour extraire le solvant.

6. Procédé de préparation de microcapsules selon la revendication 1 ou 5, **caractérisé en ce que** la température du CO₂ liquide est comprise entre 20 et 50°C et la pression entre 50 et 150.10⁵ Pa.

7. Procédé de préparation de microcapsules selon la revendication 1 ou 5, **caractérisé en ce que** la température du CO₂ supercritique est comprise entre 35 et 45°C et la pression 100 et 140.10⁵ Pa.

8. Procédé de préparation de microcapsules selon la revendication 1 ou 5 ou 7, **caractérisé en ce que** le mélange CO₂ supercritique/solvant est évacué par la phase gazeuse.

9. Procédé de préparation de microcapsules selon la revendication 1 ou 4, **caractérisé en ce que** le poids de solvant du polymère représente au moins 3 % du poids de fluide, supercritique ou liquide, utilisé pour provoquer la désolvatation du polymère, avantageusement 3,5 à 25 % du poids du fluide.

10. Procédé de préparation de microcapsules selon la revendication 1, **caractérisé en ce que** la substance active est présente une granulométrie comprise entre 0,1 et 800 µm.

11. Procédé de préparation de microcapsules selon la revendication 10, **caractérisé en ce que** la substance active est choisie dans le groupe constitué parmi les antalgiques, notamment le paracétamol, les anti-ulcéreux, les anti-hypertenseurs, les neuroleptiques, les anti-dépresseurs, les oligonucléotides, les antipyretiques, l'aspirine et ses dérivés, les anti-inflammatoires, les antibiotiques, les peptides, les vitamines.

12. Procédé de préparation de microcapsules selon l'une des revendications précédentes, **caractérisé en ce que** le procédé est mis en oeuvre dans un réacteur fermé, notamment un autoclave.

13. Microcapsules comprenant une substance active enrobée dans une couche de polymère sensiblement polaire choisie dans le groupe constitué par les polysaccharides, les dérivés cellulosiques, les polymères dérivés de l'acide acrylique ou méthacrylique, les polycyanoacrylates, les polymères biodégradables de type poly(α-hydroxyacide), les polymères dérivés des esters vinyliques, les polyesters, les polyamides, les polyanhydrides, les polyorthoesters et les polyophosphazènes, **caractérisées en ce que** les microcapsules sont susceptibles d'être obtenues par le procédé selon l'une des revendications 1 à 12.

14. Microcapsules selon la revendication 13, **caractérisées en ce que** le taux de substance active en poids par microcapsule est compris entre 25 et 95 %, avantageusement 60 à 90 %.

15. Microcapsules selon la revendication 13 ou 14, **caractérisées en ce que** le diamètre moyen est compris entre 10 nm et 1 mm.

16. Microcapsules selon l'une des revendications 13 à 15, **caractérisées en ce que** la couche de polymère polaire comprend moins de 300 ppm de solvant.

17. Application des microcapsules selon l'une des revendications 13 à 16, à la réalisation de produits cosmétiques, pharmaceutiques ou agroalimentaires.

## Patentansprüche

1. Verfahren zur Herstellung von Mikrokapseln, die eine aktive Substanz, eingehüllt in eine deutlich polare Polymerschicht, enthalten, **gekennzeichnet durch** folgende Stufen:
- Suspendieren einer aktiven Substanz in einer Lösung eines deutlich polaren Polymeren in einem organischen Lösungsmittel, wobei die Substanz in dem organischen Lösungsmittel unlöslich ist,
wobei das deutliche polare Polymere in CO₂ in flüssigem oder superkritischem Zustand unlöslich ist,
wobei das organische Lösungsmittel in flüssigem oder superkritischem CO₂ löslich ist,
- Kontaktieren der Suspension mit flüssigem oder superkritischem CO₂ **durch** Einführen von CO₂ in einen geschlossenen Reaktor, welcher bereits die Suspension enthält, derart, dass das deutlich polare Polymere unter Bedingungen nahe dem Gleichgewichtszustand, ohne plötzliche Druckänderungen (Druckminderung), kontrolliert desolvatisiert
- Gewährleisten seiner Koazervation,
- wesentliches Extrahieren des Lösungsmittels mittels CO₂ in superkritischem Zustand und Evakuieren der Mischung CO₂/Lösungsmittel,
- Gewinnen der Mikrokapseln.

2. Verfahren zur Herstellung von Mikrokapseln gemäß Anspruch 1, **dadurch gekennzeichnet, dass** das Polymere aus der Gruppe der Polysaccharide, der Zellulosederivate, der von Acrylsäure oder Methacrylsäure abgeleiteten Polymeren, der biologisch abbaubaren Polymeren vom Poly(α-hydroxysäure)-Typ, der von Vinylestern abgeleiteten Polymeren, der Polyester, der Polyamide, der Polyanhydride, der Polyorthoester, der Polycyan-acrylate und/oder der Polyphosphazene ausgewählt wird.

3. Verfahren zur Herstellung von Mikrokapseln gemäß Anspruch 2, **dadurch gekennzeichnet, dass** die Polymeren eine molare Masse zwischen 2∃310³ und 2∃310⁵ g/Mol aufweisen.

4. Verfahren zur Herstellung von Mikrokapseln gemäß Anspruch 1, **dadurch gekennzeichnet, dass** das Lösungsmittel aus der Gruppe der Ketone, Alkohole und/oder Ester ausgewählt wird.

5. Verfahren zur Herstellung von Mikrokapseln gemäß einem der Ansprüche 1 bis 4, **dadurch gekennzeichnet, dass** die Suspension mit flüssigem CO₂ kontaktiert wird, anschließend der Druck und/oder die Temperatur derart erhöht wird, dass das CO₂ in den superkritischen Zustand überführt wird, um das Lösungsmittel zu extrahieren.

6. Verfahren zur Herstellung von Mikrokapseln gemäß Anspruch 1 oder 5, **dadurch gekennzeichnet, dass** die Temperatur des flüssigen CO₂ zwischen 20 und 50 °C und der Druck zwischen 50 und 150∃10⁵ Pa liegen.

7. Verfahren zur Herstellung von Mikrokapseln gemäß Anspruch 1 oder 5, **dadurch gekennzeichnet, dass** die Temperatur des superkritischen CO₂ zwischen 35 und 45 °C und der Druck zwischen 100 und 140∃10⁵ Pa liegen.

8. Verfahren zur Herstellung von Mikrokapseln gemäß Anspruch 1 oder 5 oder 7, **dadurch gekennzeichnet, dass** die Mischung superkritisches CO₂/Lösungsmittel durch die Gasphase evakuiert wird.

9. Verfahren zur Herstellung von Mikrokapseln gemäß Anspruch 1 oder 4, **dadurch gekennzeichnet, dass** das Gewicht des Lösungsmittels des Polymeren wenigstens 3 % des Gewichts des superkritischen oder flüssigen Fluids, welches verwendet wird, um die Desolvatation des Polymeren herbeizuführen, bevorzugt 3,5 bis 25 % des Gewichts des Fluids, beträgt.

10. Verfahren zur Herstellung von Mikrokapseln gemäß Anspruch 1, **dadurch gekennzeichnet, dass** die aktive Substanz eine Granulometrie zwischen 0,1 und 800 µm aufweist.

11. Verfahren zur Herstellung von Mikrokapseln gemäß Anspruch 10, **dadurch gekennzeichnet, dass** die aktive Substanz aus der Gruppe der antalgischen Substanzen, insbesondere Paracetamol, der Antiulcermittel, der antihypertensischen Mittel, der Neuroleptika, der Antidepressiva, der Olionukleotide, der antipyretischen Substanzen, des Aspirins und seiner Derivate, der entzündungshemmenden Mittel, der Antibiotika, der Peptide und/oder der Vitamine ausgewählt wird.

12. Verfahren zur Herstellung von Mikrokapseln gemäß einem der vorhergehenden Ansprüche, **dadurch gekennzeichnet, dass** das Verfahren in einem geschlossenen Reaktor, insbesondere einem Autoklaven, durchgeführt wird.

13. Mikrokapseln, enthaltend eine aktive Substanz, eingehüllt in eine deutliche polare Polymerschicht aus Polysacchariden, Zellulosederivaten, von Acrylsäure oder Methacrylsäure abgeleiteten Polymeren, Polycyanacrylaten, biologisch abbaubaren Polymeren vom Poly(α-hydroxysäure)-Typ, von Vinylestern abgeleiten Polymeren, Polyestern, Polyamiden, Polyanhydriden, Polyorthoestern und/oder Polyophosphazenen, **dadurch gekennzeichnet, dass** die Mikrokapseln durch das Verfahren gemäß einem der Ansprüche 1 bis 12 erhältlich sind.

14. Mikrokapseln gemäß Anspruch 13, **dadurch gekennzeichnet, dass** der Gewichtsanteil der aktiven Substanz pro Mikrokapsel zwischen 25 und 95 %, bevorzugt 60 bis 90 %, beträgt.

15. Mikrokapseln gemäß Anspruch 13 oder 14, **dadurch gekennzeichnet, dass** der mittlere Durchmesser zwischen 10 nm und 1 mm beträgt.

16. Mikrokapseln gemäß einem der Ansprüche 13 bis 15, **dadurch gekennzeichnet, dass** die polare Polymerschicht wenigstens 300 ppm Lösungsmittel enthält.

17. Verwendung der Mikrokapseln gemäß einem der Ansprüche 13 bis 16 zur Herstellung kosmetischer, pharmazeutischer oder agroalimentärer Produkte.

## Claims

1. Method for preparing microcapsules comprising an active substance coated with a layer of substantially polar polymer, **characterized in that** it comprises the following stages:
- suspending an active substance in a solution of substantially polar polymer in an organic solvent, the said substance being insoluble in the organic solvent,
the said substantially polar polymer being insoluble in liquid CO₂ or CO₂ in the. supercritical state,
the said organic solvent being soluble in liquid CO₂ or supercritical CO₂,
- bringing the suspension into contact with liquid CO₂ or supercritical CO₂ by introduction of CO₂ into a closed reactor already comprising the suspension, so as to desolvate in a controlled way the substantially polar polymer under conditions close to equilibrium, without sudden variations in pressure (pressure reduction), providing its coacervation,
- substantially extracting the solvent by means of CO₂ in the supercritical state and discharging the CO₂/solvent mixture,
- recovering the microcapsules.

2. Method for preparing microcapsules according to Claim 1, **characterized in that** the polymer is chosen from the group consisting of polysaccharides, cellulose derivatives, polymers derived from acrylic or methacrylic acid, biodegradable polymers of poly(α-hydroxy acid) type, polymers derived from vinyl esters, polyesters, polyamides, polyanhydrides, polyorthoesters, polycyanoacrylates and polyphosphazenes.

3. Method for preparing microcapsules according to Claim 2, **characterized in that** the polymers exhibit a molar mass of between 2 × 10³ and 2 × 10⁵ g/mol.

4. Method for preparing microcapsules according to Claim 1, **characterized in that** the solvent is chosen from the group consisting of ketones, alcohols and esters.

5. Method for preparing microcapsules according to one of Claims 1 to 4, **characterized in that** the suspension is brought into contact with liquid CO₂ and then **in that** the pressure and/or the temperature is/are increased, so as to bring the CO₂ to the supercritical state, in order to extract the solvent.

6. Method for preparing microcapsules according to Claim 1 or 5, **characterized in that** the temperature of the liquid CO₂ is between 20 and 50°C and the pressure is between 50 × 105 and 150 × 10⁵ Pa.

7. Method for preparing microcapsules according to Claim 1 or 5, **characterized in that** the temperature of the supercritical CO₂ is between 35 and 45°C and the pressure is between 100 × 105 and 140 × 10⁵ Pa.

8. Method for preparing microcapsules according to Claim 1 or 5 or 7, **characterized in that** the supercritical CO₂/solvent mixture is discharged via the gas phase.

9. Method for preparing microcapsules according to Claim 1 or 4, **characterized in that** the weight of solvent for the polymer represents at least 3% of the weight of supercritical fluid or liquid fluid used to bring about the desolvation of the polymer, advantageously 3.5 to 25% of the weight of the fluid.

10. Method for preparing microcapsules according to Claim 1, **characterized in that** the active substance exhibits a particle size of between 0.1 and 800 µm.

11. Method for preparing microcapsules according to Claim 10, **characterized in that** the active substance is chosen from the group consisting of analgesics, in particular paracetamol, antiulceratives, antihypertensives, neuroleptics, antidepressants, oligonucleotides, antipyretics, aspirin and its derivatives, anti-inflammatories, antibiotics, peptides and vitamins.

12. Method for preparing microcapsules according to one of the preceding claims, **characterized in that** the method is carried out in a closed reactor, in particular an autoclave.

13. Microcapsules comprising an active substance coated in a layer of substantially polar polymer chosen from the group consisting of polysaccharides, cellulose derivatives, polymers derived from acrylic or methacrylic acid, polycyanoacrylates, biodegradable polymers of poly(α-hydroxy acid) type, polymers derived from vinyl esters, polyesters, polyamides, polyanhydrides, polyorthoesters and polyphosphazenes, **characterized in that** the microcapsules can be obtained by the method according to one of Claims 1 to 12.

14. Microcapsules according to Claim 13, **characterized in that** the level of active substance by weight per microcapsule is between 25 and 95%, advantageously 60 and 90%.

15. Microcapsules according to Claim 13 or 14, **characterized in that** the mean diameter is between 10 nm and 1 mm.

16. Microcapsules according to one of Claims 13 to 15, **characterized in that** the polar polymer layer comprises less than 300 ppm of solvent.

17. Application of the microcapsules according to one of Claims 13 to 16 in the preparation of cosmetic or pharmaceutical products or processed foodstuffs.
